# EUROPEAN PATENT APPLICATION

(11) **EP 4 624 040 A1**
(43) Date of publication of application: **01.10.2025**
(21) Application number: 22966200.2
(22) Date of filing: 24.11.2022
(51) Int. Cl.: B01J 8/26, C07C 2/00, C07C 5/393, C07C 15/04, C07C 15/06, C07C 15/08, C10G 35/14

(54) **DEVICE AND METHOD FOR PREPARING AROMATIC HYDROCARBONS FROM NAPHTHA**

(71) Applicant: Dalian Institute Of Chemical Physics, Chinese Academy Of Sciences, Liaoning 116023 (CN); China Shenhua Coal to Liquid and Chemical Co., Ltd, Beijing 100011 (CN)
(72) Inventor: YE, Mao, Dalian, Liaoning 116023 (CN); ZHANG, Tao, Dalian, Liaoning 116023 (CN); ZHANG, Jiming, Beijing 100011 (CN); LIU, Zhongmin, Dalian, Liaoning 116023 (CN); ZHANG, Jinling, Dalian, Liaoning 116023 (CN); YAN, Guochun, Beijing 100011 (CN); WANG, Xiangao, Dalian, Liaoning 116023 (CN); ZHANG, Yanbin, Beijing 100011 (CN); JIA, Jinming, Dalian, Liaoning 116023 (CN); WU, Qiong, Beijing 100011 (CN); TANG, Hailong, Dalian, Liaoning 116023 (CN); JIAO, Yanzhong, Beijing 100011 (CN); ZHANG, Cheng, Dalian, Liaoning 116023 (CN); MA, Xiangang, Dalian, Liaoning 116023 (CN); WANG, Jing, Dalian, Liaoning 116023 (CN); GAO, Mingbin, Dalian, Liaoning 116023 (CN); LI, Xue, Dalian, Liaoning 116023 (CN); XU, Shuliang, Dalian, Liaoning 116023 (CN); LI, Hua, Dalian, Liaoning 116023 (CN); LI, Chenggong, Dalian, Liaoning 116023 (CN)
(74) Representative: Bayramoglu et al.
(86) International application number: PCT/CN2022/134160
(87) International publication number: WO 2024/108509

(57) **Abstract**

The present application discloses a naphtha to aromatics device and method. The naphtha to aromatics device comprises a naphtha to aromatics reactor (1), a regenerator (2), and a light hydrocarbon aromatization reactor (3). In the method for producing aromatics from naphtha, a metal molecular sieve bifunctional catalyst is employed. Under the action of the catalyst in the naphtha to aromatics reactor (1), naphtha is converted into a product gas containing aromatics, light alkanes, and other components. Light alkanes and C₃, C₄, C₅ hydrocarbons separated from the product gas are further converted into aromatics and other components in the light hydrocarbon aromatization reactor (3).

## Description

### TECHNICAL FIELD

The present application relates to a fluidized bed device and a method for use thereof, and specifically relates to a device for producing aromatics from naphtha and a method thereof, belonging to the technical field of chemical engineering.

### BACKGROUND

Aromatics (benzene, toluene, and xylene, collectively referred to as BTX) are important organic chemical raw materials. Among them, para-xylene (PX) is the most noteworthy product in aromatics, mainly used to produce terephthalic acid (PTA), polyethylene terephthalate (PET), polybutylene terephthalate (PBT), and polytrimethylene terephthalate (PTT). In recent years, China's production and consumption of PX have continued to grow. In 2021, China's total PX imports amounted to approximately 13.65 million tons, with an external dependency of about 38%.

Naphtha catalytic reforming technology is the primary technical route for producing aromatics. The composition of naphtha is highly complex, as it serves not only as the main feedstock for catalytic reforming but also as a key raw material for ethylene production via cracking. The composition of naphtha plays a decisive role in the economic efficiency of the device. Generally, feedstock with high aromatic potential content and a suitable distillation range is favorable for catalytic reforming, whereas feedstock with high linear and branched aliphatic hydrocarbon content and low naphthene and aromatic content is suitable for ethylene cracking. To fully utilize naphtha resources and improve economic efficiency, it is necessary to separate linear and branched aliphatic hydrocarbons from naphthenes and aromatics in naphtha, with the former used as feedstock for ethylene production and the latter as feedstock for catalytic reforming devices.

Naphtha fractions have a wide distillation range, making it difficult for conventional separation methods to efficiently separate linear and branched aliphatic hydrocarbons from naphthenes and aromatics. Additionally, catalytic reforming technology struggles to convert linear and branched aliphatic hydrocarbons into aromatics. Naphtha feedstock for catalytic reforming typically requires distillation to remove light fractions (boiling below 60°C), thereby improving the aromatic potential content of the catalytic reforming feedstock. However, fractions with boiling points above 60°C still contain significant amounts of linear and branched aliphatic hydrocarbons that are difficult to convert into aromatics. Therefore, the high-selectivity conversion of linear and branched aliphatic hydrocarbons into aromatics has been a key focus and challenge in the development of naphtha-to-aromatics technology.

Due to thermodynamic equilibrium limitations, para-xylene accounts for only ~24% of the xylene mixture produced by naphtha catalytic reforming devices, necessitating further para-xylene production through isomerization-separation processes. Thus, increasing the para-xylene content in the xylene mixture is an important approach to reducing energy consumption in para-xylene production.

### SUMMARY

To achieve the preparation of aromatics from naphtha with low aromatic potential content, improve the para-xylene content in mixed xylenes, and reduce production energy consumption, the present application provides a device for producing aromatics from naphtha and a method for producing aromatics from naphtha.

According to one aspect of the present application, a device for producing aromatics from naphtha is provided, comprising a naphtha to aromatics reactor, a regenerator, and a light hydrocarbon aromatization reactor.

The device for producing aromatics from naphtha, wherein the device for producing aromatics from naphtha comprises a naphtha to aromatics reactor, a regenerator, and a light hydrocarbon aromatization reactor;
the naphtha to aromatics reactor is connected to the regenerator via a spent catalyst delivery pipe I; the regenerator is connected to the naphtha to aromatics reactor via a regenerated catalyst delivery pipe;
the light hydrocarbon aromatization reactor includes a riser reactor, and the riser reactor is connected to a bed reactor; and
the regenerator is connected to the riser reactor via a regenerated catalyst slide valve II; the bed reactor is connected to the regenerator via a spent catalyst delivery pipe II.

Optionally, a gas-solid separation zone is provided on the upper part of the naphtha to aromatics reactor; a product gas delivery pipe I is provided in the gas-solid separation zone; and
a naphtha to aromatics reaction zone is provided at the bottom of the naphtha to aromatics reactor; the regenerated catalyst delivery pipe inputs a catalyst into the naphtha to aromatics reaction zone; a naphtha to aromatics reactor distributor for introducing a naphtha raw material is provided at the bottom of the naphtha to aromatics reaction zone;
Optionally, a gas-solid separation unit I and a gas collection chamber I are provided in the naphtha to aromatics reactor; the gas collection chamber I is located at the top of the gas-solid separation zone; a gas outlet of the gas-solid separation unit I is connected to the gas collection chamber I, and the gas collection chamber I is connected to the product gas delivery pipe I;
Optionally, a stripper I is provided beneath the naphtha to aromatics reaction zone; the naphtha to aromatics reaction zone is connected to the spent catalyst delivery pipe I via the stripper I;
Optionally, the stripper I is connected to the spent catalyst delivery pipe I via the spent catalyst slide valve I;
Optionally, the gas-solid separation unit I adopts one or more groups of gas-solid cyclone separators, and each group of gas-solid cyclone separators includes a first-stage gas-solid cyclone separator and a second-stage gas-solid cyclone separator.

Optionally, a regenerator gas-solid separation zone is provided on upper part of the regenerator; a flue gas delivery pipe is provided in the regenerator gas-solid separation zone;
a regeneration zone is provided at the lower part of the regenerator; the spent catalyst delivery pipe I and the spent catalyst delivery pipe II are used to feed the spent catalyst into the regeneration zone; a regenerator distributor for introducing a regeneration gas is provided at lower part of the regenerator;
the regenerated catalyst delivery pipe delivers a regenerated catalyst in the regeneration zone to the naphtha to aromatics reactor; the regenerated catalyst slide valve II delivers the regenerated catalyst to the riser reactor;
Optionally, a regenerator gas-solid separation unit and a regenerator gas collection chamber are provided in the regenerator shell; the regenerator gas collection chamber is located at the top of the regenerator gas-solid separation zone; a gas outlet of the regenerator gas-solid separation unit is connected to the regenerator gas collection chamber; the regenerator gas collection chamber is connected to the flue gas delivery pipe.

Optionally, a regenerator stripper is provided beneath the regeneration zone; the regeneration zone is connected to the regenerated catalyst slide valve I and the regenerated catalyst slide valve II via the regenerator stripper;
Optionally, the regenerated catalyst slide valve I is connected to the naphtha to aromatics reactor via the regenerated catalyst delivery pipe; the regenerated catalyst slide valve II is connected to the riser reactor;
Optionally, the regenerator gas-solid separation unit adopts one or more groups of gas-solid cyclone separators, and each group of gas-solid cyclone separators includes a first-stage gas-solid cyclone separator and a second-stage gas-solid cyclone separator.

Optionally, a bed reactor gas-solid separation zone is provided on upper part of the bed reactor; a product gas delivery pipe II is provided in the bed reactor gas-solid separation zone;
a light hydrocarbon aromatization reaction zone is provided at the lower part of the bed reactor; a bed reactor distributor is provided at the lower part of the light hydrocarbon aromatization reaction zone; the bed reactor distributor is used to feed a bed reactor feedstock; upper end of the riser reactor penetrates bottom of the bed reactor and is inserted into the bed reactor along axial direction;
the regenerated catalyst slide valve II delivers a regenerated catalyst to a feed inlet end of the riser reactor;
Optionally, a gas-solid separation unit II and a gas collection chamber II are provided in the bed reactor gas-solid separation zone; a gas outlet of the gas-solid separation unit II is connected to the gas collection chamber II; a catalyst outlet of the gas-solid separation unit II is located in the light hydrocarbon aromatization reaction zone; the gas collection chamber II is connected to a product gas delivery pipe II located outside the bed reactor;
Optionally, the light hydrocarbon aromatization reaction zone is connected to a stripper II, and the bed reactor is connected to the spent catalyst delivery pipe II via the stripper II;
Optionally, the stripper II is connected to the spent catalyst delivery pipe II via the spent catalyst slide valve II;
Optionally, the gas-solid separation unit II is a gas-solid cyclone separator; and the catalyst outlet of the gas-solid separation unit II is located above outlet end of the riser reactor.

The present application also provides a method for preparing aromatics from naphtha, using the abovementioned device; a metal molecular sieve bifunctional catalyst is used as a catalyst;
feeding a raw material containing naphtha into the naphtha to aromatics reactor; feeding a riser reactor feedstock into the riser reactor; feeding the bed reactor feedstock containing C₃, C₄ and C₅ hydrocarbons into the bed reactor; feeding a regeneration gas to the regenerator;
the naphtha to aromatics reactor and the bed reactor output a product gas, and a spent catalyst is input into the regenerator via the spent catalyst delivery pipe I and the spent catalyst delivery pipe II; after the spent catalyst reacts with the regeneration gas in the regenerator to be regenerated, it is fed into the naphtha to aromatics reactor and the riser reactor; the regenerator discharges a flue gas.

Optionally, the catalyst is a metal-modified HZSM-5 zeolite molecular sieve; a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

Optionally, the naphtha is at least one selected from the group consisting of coal direct liquefaction naphtha, coal indirect liquefaction naphtha, straight-run naphtha, and hydrocracked naphtha;
Optionally, the raw material containing naphtha further comprises unconverted naphtha separated from the product gas flow; the unconverted naphtha contains linear aliphatic hydrocarbons, branched aliphatic hydrocarbons, and naphthenes of C₄-C₁₂;
Optionally, the reaction conditions in the naphtha to aromatics reaction zone are: gas superficial velocity in a range from 0.5 m/s to 2.0 m/s, reaction temperature in a range from 500°C to 650°C, reaction pressure in a range from 100 kPa to 500 kPa, bed density in a range from 150 kg/m³ to 700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among 500°C, 520°C, 530°C, 540°C, 550°C, 560°C, 570°C, 580°C, 590°C, 600°C, 610°C, 620°C, 630°C, 640°C, 650°C or any range between two values.

Optionally, the reaction pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Optionally, the carbon content in the regenerated catalyst is ≤0.5 wt%;
Optionally, the regeneration gas is at least one selected from the group consisting of oxygen, air, and oxygen-enriched air;
Optionally, the regeneration conditions in the regeneration zone are: gas superficial velocity in a range from 0.5-2.0 m/s, regeneration temperature in a range from 600°C to 750°C, regeneration pressure in a range from 100 KPa to 500 kPa, bed density in a range from 150 kg/m³ to 700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the regeneration temperature is independently selected from any value among 600°C, 615°C, 630°C, 645°C, 670°C, 685°C, 700°C, 715°C, 730°C, 745°C, 750°C or any range between two values.

Optionally, the regeneration pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Optionally, the riser reactor feedstock further comprises water vapor; the water vapor content in the riser reactor feedstock is in a range from 0 wt% to 80 wt%;
Optionally, the light alkanes in the riser reactor feedstock are separated from the product gas flow;
Optionally, the process conditions for the riser reactor are: gas superficial velocity in a range from 3.0 m/s to 10.0 m/s, temperature in a range from 580 to 700°C, pressure in a range from 100 to 500 kPa, bed density in a range from 50 kg/m³ to 150 kg/m³;
Optionally, the gas superficial velocity is independently selected from any value among 3.0m/s, 3.5m/s, 4.0m/s, 4.5m/s, 5.0m/s, 5.5m/s, 6.0m/s, 6.5m/s, 7.0m/s, 7.5m/s, 8.0m/s, 8.5m/s, 9.0m/s, 9.5m/s, 10.0m/s or any range between two values.

Optionally, the temperature is independently selected from any value among 580°C, 590°C, 600°C, 610°C, 620°C, 630°C, 640°C, 650°C, 660°C, 670°C, 680°C, 690°C, 700°C or any range between two values.

Optionally, the pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 50kg/m³, 60kg/m³, 70kg/m³, 80kg/m³, 90kg/m³, 100kg/m³, 110kg/m³, 120kg/m³, 130kg/m³, 140kg/m³, 150kg/m³ or any range between two values.

Optionally, the bed reactor feedstock comprises at least one selected from the group consisting of C₃, C₄, and Cs hydrocarbons;
Optionally, the C₃, C₄, and Cs hydrocarbons are separated from the product gas flow;
Optionally, the bed reactor feedstock comprises at least one selected from the group consisting of C₄ and C₅ hydrocarbons;
Optionally, the C₄ and C₅ hydrocarbons are separated from the product gas flow;
Optionally, the process conditions for the light hydrocarbon aromatization reaction zone are: gas superficial velocity in a range from 0.5 m/s to 2.0 m/s, reaction temperature in a range from 550°C to 665°C, reaction pressure in a range from 100 kPa to 500 kPa, bed density in a range from 150 kg/m³ to 700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among550°C, 560°C, 570°C, 580°C, 590°C, 600°C, 610°C, 620°C, 630°C, 640°C, 650°C, 660°C, 665°C or any range between two values.

Optionally, the reaction pressure is independently selected from any value among100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Optionally, the method comprises: introducing naphtha into the naphtha to aromatics reaction zone of the naphtha to aromatics reactor via the distributor of naphtha to aromatics reactor, where it contacts with a catalyst from the regenerator to generate a product gas flow containing BTX, light olefins, hydrogen, light alkanes, combustible gas, heavy aromatics, and unconverted naphtha, while the catalyst becomes coked and converts to the spent catalyst;
feeding the regeneration gas into the regeneration zone of the regenerator via the reactor distributor, where it contacts with the spent catalyst from the naphtha to aromatics reactor and the spent catalyst from the light hydrocarbon aromatization reactor, reacting with coke on the spent catalyst to generate flue gas and the spent catalyst converts to the regenerate the catalyst;
feeding the riser reactor feedstock into the feed inlet end of the riser reactor, which contacts with the regenerated catalyst from the regenerator, converting under catalytic action into a flow containing BTX, light olefins, and H₂, which then enters the lower part of the light hydrocarbon aromatization reaction zone in the bed reactor via the outlet end of the riser reactor;
feeding the bed reactor feedstock into the light hydrocarbon aromatization reaction zone via the reactor distributor, where it contacts with a catalyst from the riser reactor to generate light hydrocarbon aromatization product gas flow containing BTX, light olefins, and H₂, while the catalyst becomes coked and converts to the spent catalyst.

Optionally, the product gas flow enters the gas-solid separation unit I to remove entrained spent catalyst, then enters the gas collection chamber I, and is conveyed to downstream sections via the product gas delivery pipe I.

The spent catalyst in the naphtha to aromatics reaction zone enters the stripper I through the open end of the stripper I inlet pipe, undergoes stripping, and is then transported to downstream sections via the spent catalyst slide valve I and the spent catalyst delivery pipe I.

Specifically, the downstream section is the regenerator.

The term "light olefins" refers to ethylene and propylene.

The term "light alkanes" refers to ethane and propane.

The term "combustible gas" includes methane and CO.

The term "heavy aromatics" refers to aromatic hydrocarbons with nine or more carbon atoms per molecule.

Optionally, the method includes: the spent catalyst from the naphtha to aromatics reaction zone sequentially passes through the stripper I, the spent catalyst slide valve I, and the spent catalyst delivery pipe I into the regenerator, where it contacts and reacts with the regeneration gas to produce flue gas and regenerated catalyst;

Optionally, the method includes: the spent catalyst from the light hydrocarbon aromatization reaction zone sequentially passes through the stripper II, the spent catalyst slide valve II, and the spent catalyst delivery pipe II into the regenerator, where it contacts and reacts with the regeneration gas to produce flue gas and regenerated catalyst;
the flue gas enters the regenerator gas-solid separation unit to remove entrained regenerated catalyst, then enters the regenerator gas collection chamber, and is conveyed to downstream sections via the flue gas delivery pipe.

Optionally, the regenerated catalyst sequentially passes through the regenerator stripper, the regenerated catalyst slide valve I, and the regenerated catalyst delivery pipe into the naphtha to aromatics reactor.

Optionally, the regenerated catalyst sequentially passes through the regenerator stripper and the regenerated catalyst slide valve II into the light hydrocarbon aromatization reactor.

Optionally, the light hydrocarbon aromatization product gas enters the gas-solid separation unit II to remove entrained catalyst, then enters the gas collection chamber II, and is conveyed to downstream sections via the product gas delivery pipe II.

The catalyst in the light hydrocarbon aromatization reaction zone passes through the stripper II, the spent catalyst slide valve II, and the spent catalyst delivery pipe II into downstream sections.

Specifically, the downstream section is the regenerator.

In the present application, the components of the naphtha include C₄-C₁₂ linear aliphatic hydrocarbons, branched aliphatic hydrocarbons, naphthenes, and aromatics.

In the present application, the term "aromatics" refers to benzene, toluene, and xylene, collectively termed BTX.

In the method of the present application, the naphtha feedstock has an aromatics potential content of 0-80 wt%, with a single-pass conversion rate of 70-95 wt%. Unconverted naphtha is separated from the product gas and recycled as feedstock to the naphtha to aromatics reactor. A portion of light alkanes is separated from the product gas and recycled as feedstock to the riser reactor in the light hydrocarbon aromatization reactor. C₃, C₄, and Cs hydrocarbons are separated from the product gas and recycled as feedstock to the bed reactor in the light hydrocarbon aromatization reactor. The final product distribution is: 60-75 wt% BTX, 6-14 wt% light olefins, 3-7 wt% hydrogen, 3-8 wt% light alkanes, 4-6 wt% combustible gas, 4-8 wt% heavy aromatics, and 0.5-1 wt% coke. The para-xylene content in the mixed xylene product exceeds 50-65 wt%.

The beneficial effects of the present application include:
(1) The method for preparing aromatics from naphtha described in the present application can convert linear and branched aliphatic hydrocarbons into aromatics with high selectivity. Therefore, the method described in the present application has a wide range of raw material adaptability, and aromatics can be prepared using naphtha with a low potential aromatic content as a raw material.
(2) By employing the light hydrocarbon aromatization reactor and a metal molecular sieve bifunctional catalyst, the method achieves aromatization of light alkanes, C₄, and Cs hydrocarbons, significantly improving the overall aromatics yield in naphtha-to-aromatics technology.
(3) The para-xylene content in the xylene mixture produced exceeds 50 wt%, far surpassing the thermodynamic equilibrium content (approximately 24 wt%). This enhances para-xylene yield while drastically reducing separation energy consumption.
(4) The naphtha-to-aromatics device in the present application is provided with a naphtha-to-aromatics reactor and a light hydrocarbon aromatization reactor. Light alkanes, being highly stable, require higher reaction temperatures. In this device, the light hydrocarbon aromatization reactor operates at a higher temperature than the naphtha-to-aromatics reactor. This configuration ensures efficient aromatization of light alkanes, C₄, and Cs hydrocarbons in the high-temperature reactor (boosting reaction rates and aromatics yield) while minimizing coking of naphtha in the lower-temperature reactor. The naphtha-to-aromatics device in the present application achieves the beneficial effects of reducing the yield of light alkanes and increasing the yield of aromatics by separately setting up a high-temperature light hydrocarbon aromatization reactor and a relatively low-temperature naphtha-to-aromatics reactor.

### BRIEF DESCRIPTION OF THE DRAWINGS

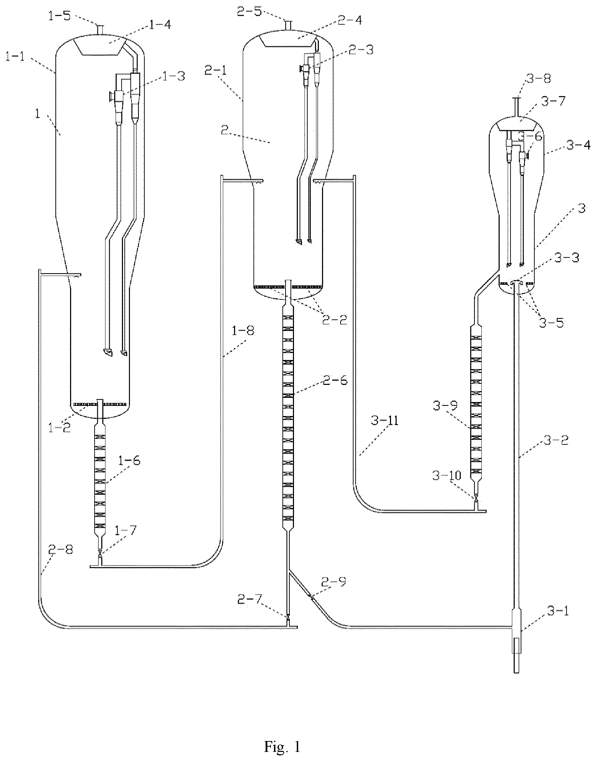

Fig. 1 is a schematic diagram of a naphtha to aromatics device according to one embodiment of the present application.

List of Components and Reference Numerals:
1 naphtha to aromatics reactor;
   1-1 naphtha to aromatics reactor shell;
   1-2 naphtha to aromatics reactor distributor;
   1-3 gas-solid separation unit I;
   1-4 gas collection chamber I;
   1-5 product gas delivery pipe I;
   1-6 stripper I;
   1-7 spent catalyst slide valve I;
   1-8 spent catalyst delivery pipe I.
2 regenerator;
   2-1 regenerator shell;
   2-2 regenerator distributor;
   2-3 regenerator gas-solid separation unit;
   2-4 regenerator gas collection chamber;
   2-5 flue gas delivery pipe;
   2-6 regenerator stripper;
   2-7 regenerated catalyst slide valve I;
   2-8 regenerated catalyst delivery pipe;
   2-9 regenerated catalyst slide valve II.
3 light hydrocarbon aromatization reactor;
   3-1 inlet end of the riser reactor;
   3-2 middle section of the riser reactor;
   3-3 outlet end of the riser reactor;
   3-4 bed reactor shell;
   3-5 bed reactor distributor;
   3-6 gas-solid separation unit II;
   3-7 gas collection chamber II;
   3-8 product gas delivery pipe II;
   3-9 stripper II;
   3-10 spent catalyst slide valve II;
   3-11 spent catalyst delivery pipe II.

### DETAILED DESCRIPTION OF THE EMBODIMENTS

The present application will be described in detail below with reference to examples, but the present application is not limited to these examples.

The following describes possible embodiments.

The present application provides a device for producing aromatics from naphtha, as shown in Fig. 1. The device comprises a naphtha to aromatics reactor 1, a regenerator 2, and a light hydrocarbon aromatization reactor 3.

The naphtha to aromatics reactor 1 includes: naphtha to aromatics reactor shell 1-1, naphtha to aromatics reactor distributor 1-2, gas-solid separation unit I 1-3, gas collection chamber I 1-4, product gas delivery pipe I 1-5, stripper I 1-6; spent catalyst slide valve I 1-7, spent catalyst delivery pipe I 1-8.

The naphtha to aromatics reactor shell 1-1 comprises a naphtha to aromatics reactor upper shell and a naphtha to aromatics reactor lower shell. The naphtha to aromatics reactor upper shell encloses a gas-solid separation zone and the naphtha to aromatics reactor lower shell encloses a naphtha to aromatics reaction zone. The naphtha to aromatics reactor shell is provided with an outlet of the regenerated catalyst delivery pipe 2-8.

The naphtha to aromatics reaction zone is equipped with the distributor 1-2 at its lower section for introducing naphtha feedstock.

The naphtha to aromatics reactor shell 1-1 houses the gas-solid separation unit I 1-3 and gas collection chamber I 1-4; The chamber I 1-4, located at the naphtha to aromatics reactor shell's inner top, connects to the gas outlet of the gas-solid separation unit I 1-3 and the product gas delivery pipe I 1-5. The catalyst outlet of separation unit I 1-3 is positioned above the open end of the stripper I 1-6 inlet pipe.

The stripper I 1-6 is located below the naphtha to aromatics reaction zone; the inlet of the stripper I 1-6 is located inside the naphtha to aromatics reactor shell 1-1; and the outlet of the stripper I 1-6 located outside the naphtha to aromatics reactor shell 1-1 and is connected to the spent catalyst slide valve I 1-7; the open end of the inlet of the stripper I 1-6 is located above the naphtha to aromatics reactor distributor 1-2.

The spent catalyst slide valve I 1-7 is provided below the stripper I 1-6; the inlet of the spent catalyst slide valve I 1-7 is connected to the outlet of the stripper I 1-6, the outlet of the spent catalyst slide valve I 1-7 is connected to the inlet of the spent catalyst delivery pipe I 1-8, and the outlet of the spent catalyst delivery pipe I 1-8 is connected to the regenerator shell 2-1.

The spent catalyst slide valve I 1-7 is used to control the circulation amount of the spent catalyst.

In a preferred embodiment, the gas-solid separation unit I 1-3 uses one or more groups of gas-solid cyclone separators, and each group of gas-solid cyclone separators includes a first-stage gas-solid cyclone separator and a second-stage gas-solid cyclone separator.

The regenerator 2 comprises: the regenerator shell 2-1, the regenerator distributor 2-2, the regenerator gas-solid separation unit 2-3, the regenerator gas collection chamber 2-4, the flue gas delivery pipe 2-5, the regenerator stripper 2-6, the regenerated catalyst slide valve I 2-7, the regenerated catalyst delivery pipe 2-8, and the regenerated catalyst slide valve II 2-9.

The regenerator shell 2-1 comprises the regenerator upper shell and the regenerator lower shell, wherein the regenerator upper shell encloses the gas-solid separation zone, and the regenerator lower shell encloses the regeneration zone; the regenerator shell 2-1 is provided with an outlet of the spent catalyst delivery pipe I 1-8 and the outlet of the spent catalyst delivery pipe II 3-11.

The regenerator distributor 2-2 is provided at the lower part of the regeneration zone, and the regenerator distributor 2-2 is used for introducing the regeneration gas.

The regenerator shell 2-1 is also provided with a regenerator gas-solid separation unit 2-3 and a regenerator gas collection chamber 2-4; the regenerator gas collection chamber 2-4 is located at the inner top of the regenerator shell 2-1; the gas outlet of the regenerator gas-solid separation uint 2-3 is connected to the regenerator gas collection chamber 2-4; the regenerator gas collection chamber 2-4 is connected to the flue gas delivery pipe 2-5; the catalyst outlet end of the regenerator gas-solid separation unit 2-3 is located above the opening end of the inlet pipe of the regenerator stripper 2-6.

The regenerator stripper 2-6 is provided below the regeneration zone; the inlet of the regenerator stripper 2-6 is located inside the regenerator shell 2-1; the outlet of the regenerator stripper 2-6 is located outside the regenerator shell 2-1, and is connected to the regenerated catalyst slide valve I 2-7 and the regenerated catalyst slide valve II 2-9; the opening end of the inlet of the regenerator stripper 2-6 is located above the regenerator distributor 2-2.

The regenerated catalyst slide valve I 2-7 is connected to the inlet of the regenerated catalyst delivery pipe 2-8, and the outlet of the regenerated catalyst delivery pipe 2-8 is connected to the naphtha to aromatics reactor shell 1-1. The regenerated catalyst slide valve I 2-7 is used to control the circulation amount of the regenerated catalyst.

The regenerated catalyst slide valve II 2-9 is used to control the circulation amount of the regenerated catalyst.

In a preferred embodiment, the regenerator gas-solid separation unit 2-3 uses one or more groups of gas-solid cyclone separators, and each group of gas-solid cyclone separators includes the first-stage gas-solid cyclone separator and the second-stage gas-solid cyclone separator.

The light hydrocarbon aromatization reactor 3 comprises: the inlet end of the riser reactor 3-1, the middle part of the riser reactor 3-2, the outlet end of the riser reactor 3-3, the bed reactor shell 3-4, the bed reactor distributor 3-5, the gas-solid separation unit II 3-6, the gas collection chamber II 3-7, the product gas delivery pipe II 3-8, the stripper II 3-9, the spent catalyst slide valve II 3-10, and the spent catalyst delivery pipe II 3-11.

The bed reactor shell 3-4 comprises an upper shell of the bed reactor and a lower shell of the bed reactor, the upper shell of the bed reactor encloses a gas-solid separation zone, and the lower shell of the bed reactor encloses a light hydrocarbon aromatization reaction zone; the bed reactor distributor 3-5 is provided at the inner lower part of the light hydrocarbon aromatization reaction zone; the upper section of the riser reactor penetrates the bottom of the bed reactor and is axially inserted in the bed reactor; the outlet end of the riser reactor 3-3 is located at the inner lower part of the light hydrocarbon aromatization reaction zone.

The gas-solid separation zone of the bed reactor is provided with the gas-solid separation unit II 3-6 and a gas collection chamber II 3-7; the gas outlet of the gas-solid separation unit II 3-6 is connected to the gas collection chamber II 3-7; the catalyst outlet of the gas-solid separation unit II 3-6 is located in the light hydrocarbon aromatization reaction zone; the gas collection chamber II 3-7 is connected to the product gas delivery pipe II 3-8 located outside the bed reactor.

The stripper II 3-9 and the spent catalyst slide valve II 3-10 are provided outside the shell of the bed reactor; the inlet of the stripper II 3-9 is located at the lower shell of the bed reactor; the outlet of the stripper II 3-9 is connected to the inlet of the spent catalyst slide valve II 3-10, the outlet of the spent catalyst slide valve II 3-10 is connected to the inlet of the spent catalyst delivery pipe II 3-11, and the outlet of the spent catalyst delivery pipe II 3-11 is connected to the regenerator shell 2-1.

In a preferred embodiment, the gas-solid separation unit II 3-6 is a gas-solid cyclone separator; the catalyst outlet of the gas-solid separation unit II 3-6 is located above the outlet end of the riser reactor 3-3.

In a preferred embodiment, a gas collection chamber II 3-7 is provided at the inner top of the bed reactor.

In a preferred embodiment, the bed reactor distributor 3-5 is used to feed the bed reactor feedstock.

In a preferred embodiment, the inlet end of the riser reactor 3-1 is connected to the regenerated catalyst slide valve II 2-9 through a pipeline.

In a preferred embodiment, the inlet end of the riser reactor 3-1 is used to feed the catalyst and the riser reactor feedstock.

In order to achieve aromatization of liner and branched aliphatic hydrocarbons, increase the yield of aromatic hydrocarbons and the content of p-xylene in mixed xylenes, the present application provides a method for preparing aromatic hydrocarbons from naphtha, the method using any of the above-mentioned devices and a metal molecular sieve bifunctional catalyst;

The catalyst adopts metal-modified HZSM-5 zeolite molecular sieve; the metal used for metal modification is at least one selected from the group consisting of La, Zn, Ga, Fe, Mo, and Cr;
the metal modification method comprises: placing the HZSM-5 zeolite molecular sieve in a metal salt solution, impregnating, drying and calcining to obtain the metal modified HZSM-5 zeolite molecular sieve.

The method comprises the following steps:
a) naphtha enters the naphtha to aromatics reaction zone of the naphtha to aromatics reactor 1 through the naphtha to aromatics reactor distributor 1-2, contacts with the catalyst from the regenerator 2, generates a product gas flow containing BTX, light olefins, hydrogen, light alkanes, combustible gas, heavy aromatics and unconverted naphtha, and at the same time, the catalyst is coked and converted into the spent catalyst; the product gas flow enters the gas-solid separation unit I 1-3 to remove the spent catalyst carried therein, then enters the gas collection chamber I 1-4, and enters the downstream section through the product gas delivery pipe I 1-5; the spent catalyst in the naphtha to aromatics reaction zone enters the stripper I 1-6 through the open end of the inlet pipe of the stripper I 1-6, is stripped, and after stripping, enters the regenerator 2 through the spent catalyst slide valve I 1-7 and the spent catalyst delivery pipe I 1-8;
b) passing the regenerated gas into the regeneration zone of the regenerator 2 through the regenerator distributor 2-2, and contacting with the spent catalyst, the coke on the spent catalyst reacts with the regenerated gas to generate flue gas, and at the same time, the spent catalyst is converted into the regenerated catalyst; the flue gas enters the regenerator gas-solid separation unit 2-3 to remove the regenerated catalyst carried therein, and then enters the regenerator gas collection chamber 2-4, and enters the downstream section through the flue gas delivery pipe 2-5; the regenerated catalyst passes through the regenerator stripper 2-6, the regenerated catalyst slide valve I 2-7 and the regenerated catalyst delivery pipe 2-8 in sequence to enter the naphtha to aromatics reactor 1; the regenerated catalyst passes through the regenerator stripper 2-6 and the regenerated catalyst slide valve II 2-9 in sequence to enter the light hydrocarbon aromatization reactor 3;
c) feeding the riser reactor feedstock into the riser reactor through the inlet end of the riser reactor 3-1, contacting and reacting with the regenerated catalyst from the regenerator, and converting the riser reactor feedstock into a flow containing components such as BTX, light olefins and H₂ under the action of the catalyst, and then entering the inner lower part of the light hydrocarbon aromatization reaction zone in the bed reactor through the outlet end of the riser reactor 3-3; feeding the bed reactor feedstock into the light hydrocarbon aromatization reaction zone through the bed reactor distributor 3-5, contacting with the catalyst from the riser reactor, and generating a light hydrocarbon aromatization product gas containing components such as BTX, light olefins and H₂; the light hydrocarbon aromatization product gas enters the gas-solid separation unit II 3-6 to remove the catalyst carried therein, and then enters the gas collection chamber II 3-7, and then enters the downstream section through the product gas delivery pipe II 3-8; the catalyst in the light hydrocarbon aromatization reaction zone passes through the stripper II 3-9, the spent catalyst slide valve II 3-10 and the spent catalyst delivery pipe II 3-11 to enter the regenerator 2.

The light olefins are ethylene and propylene.

The light alkanes are ethane and propane.

The combustible gas includes methane, CO and the like.

The heavy aromatic hydrocarbons refer to aromatic hydrocarbons having 9 or more carbon atoms in the molecule.

In a preferred embodiment, the naphtha is selected from at least one of coal direct liquefaction naphtha, coal indirect liquefaction naphtha, straight run naphtha and hydrocracked naphtha.

In a preferred embodiment, the naphtha further comprises unconverted naphtha separated from the product gas flow.

In a preferred embodiment, the process conditions of the naphtha to aromatics reaction zone are: gas superficial velocity of 0.5-2.0 m/s, reaction temperature of 500-650 °C, reaction pressure of 100-500 kPa, and bed density of 150-700 kg/m³.

In a preferred embodiment, the carbon content in the regenerated catalyst is ≤0.5 wt%.

In a preferred embodiment, the regeneration gas is selected from at least one of oxygen, air and oxygen enriched air.

In a preferred embodiment, the process conditions in the regeneration zone are: gas superficial linear velocity is 0.5-2.0 m/s, regeneration temperature is 600-750 °C, regeneration pressure is 100-500 kPa, and bed density is 150-700 kg/m³.

In a preferred embodiment, the riser reactor feedstock comprises water vapor and light alkanes separated from the product gas flow.

In a preferred embodiment, the water vapor content in the riser reactor feed is 0-80 wt%.

In a preferred embodiment, the process conditions of the riser reactor are: gas superficial velocity of 3.0-10.0 m/s, temperature of 580-700°C, pressure of 100-500 kPa, and bed density of 50-150 kg/m³.

In a preferred embodiment, the bed reactor feedstock comprises C₃, C₄ and C₅ hydrocarbons.

In a preferred embodiment, the bed reactor feedstock comprises C₄ and C₅ hydrocarbons.

In a preferred embodiment, the C₃, C₄ and C₅ hydrocarbons are derived from C₃, C₄ and C₅ hydrocarbons separated from the product gas flow.

In a preferred embodiment, the C₄ and C₅ hydrocarbons are derived from C₄ and C₅ hydrocarbons separated from the product gas flow.

The C₃, C₄ and C₅ hydrocarbons refer to hydrocarbons with 3, 4 and 5 carbon atoms.

The C₄ and C₅ hydrocarbons refer to hydrocarbons with 4 and 5 carbon atoms.

In a preferred embodiment, the process conditions of the light hydrocarbon aromatization reaction zone are: gas superficial linear velocity of 0.5-2.0 m/s, reaction temperature of 550-665 °C, reaction pressure of 100-500 kPa, and bed density of 150-700 kg/m³.

In the embodiment described in the present application, the naphtha feedstock has a latent aromatic content of 0-80wt%, the single-pass conversion rate of naphtha is 70-95wt%, the unconverted naphtha is separated from the product gas and returned to the naphtha to aromatics reactor 1 as a raw material, some light alkanes are separated from the product gas and returned to the riser reactor in the light hydrocarbon aromatization reactor 3 as a raw material, C₃, C₄ and C₅ hydrocarbons are separated from the product gas and returned to the bed reactor in the light hydrocarbon aromatization reactor 3 as a raw material, and the final product distribution is: 60-75wt% BTX, 6-14wt% light olefins, 3-7wt% hydrogen, 3-8wt% light alkanes, 4-6wt% combustible gas, 4-8wt% heavy aromatics, and 0.5-1wt% coke. The content of p-xylene in the mixed xylene in the product is 50-65 wt%.

The catalyst used in the following Examples was prepared as follows:
100 g of HZSM-5 zeolite molecular sieve (manufactured by Nankai University Catalyst Plant, Si/Al = 15) was immersed in a 10 wt% zinc nitrate aqueous solution. The mass ratio of HZSM-5 zeolite to the zinc nitrate solution (solid-to-liquid ratio) was 1:10. The mixture was impregnated at 80°C for 6 hours, filtered, dried at 120°C under air atmosphere for 4 hours, and then calcined at 550°C under air atmosphere for 4 hours to obtain a [Zn]HZSM-5 molecular sieve sample.

100 g of the [Zn]HZSM-5 molecular sieve sample was mixed with an amorphous binder containing aluminum or silicon. Detailed steps:
The [Zn]HZSM-5 sample, pseudo-boehmite, silica sol, xanthan gum (bio-gum), and water were uniformly mixed. The mixture underwent slurrying, homogenization, and deaeration to form a slurry. The weight fractions of the slurry components were:

| | |
|---|---|
| [Zn]HZSM-5 | 35 parts by weight |
| Al₂O₃ | 20 parts by weight |
| SiO₂ | 45 parts by weight |
| H₂O | 240 parts by weight |
| xanthan gum | 1 parts by weight |

The slurry was spray-dried to form microsphere particles with a particle size distribution of 20 to 100 µm. The particles were calcined in a muffle furnace at 550°C for 3 hours, yielding a [Zn]HZSM-5 formed molecular sieve with an attrition index of 1.2.

### Example 1

This embodiment employs the device illustrated in Fig. 1.

Naphtha feedstock fed to the naphtha to aromatics reactor is coal direct liquefaction naphtha with an aromatics potential content of 78 wt%, including unconverted naphtha recycled from the separated product gas flow.

Naphtha to aromatics reaction zone conditions: gas superficial velocity is 0.5 m/s, reaction temperature is 645°C, reaction pressure is 100 kPa, bed density is 700 kg/m³.

Regeneration gas is air.

Regeneration zone conditions in the regenerator: gas superficial velocity is 0.5 m/s, regeneration temperature is 745°C, regeneration pressure is 100 kPa, bed density is 700 kg/m³.

Carbon content in the regenerated catalyst is 0.2 wt%.

Riser reactor feedstock is light alkanes separated from the product gas flow.

Riser reactor conditions: gas superficial velocity is 3.0 m/s, temperature is 690°C, pressure is 100 kPa, bed density is 150 kg/m³.

Bed reactor feedstock is C₃, C₄, and Cs hydrocarbons separated from the product gas flow.

Light hydrocarbon aromatization reaction zone conditions: gas superficial velocity is 0.5 m/s, reaction temperature is 665°C, reaction pressure is 100 kPa, bed density is 700 kg/m³.

Single-pass conversion of naphtha feedstock into the naphtha to aromatics reactor is 70.3 wt%.

Product distribution: 74.6 wt% BTX, 6 wt% light olefins, 3 wt% hydrogen, 3.5 wt% light alkanes, 5.1 wt% combustible gas, 7 wt% heavy aromatics, 0.8 wt% coke. The content of p-xylene in the mixed xylene in the product is 50.5 wt%.

### Example 2

This embodiment employs the device illustrated in Fig. 1.

Naphtha feedstock fed to the naphtha to aromatics reactor is coal direct liquefaction naphtha with an aromatics potential content of 0.1 wt%, including unconverted naphtha recycled from the separated product gas flow.

Naphtha to aromatics reaction zone conditions: gas superficial velocity is 2.0 m/s, reaction temperature is 510°C, reaction pressure is 500 kPa, bed density is 150 kg/m³.

Regeneration gas is oxygen.

Regeneration zone conditions in the regenerator: gas superficial velocity is 2.0 m/s, regeneration temperature is 610°C, regeneration pressure is 500 kPa, bed density is 150 kg/m³.

Carbon content in the regenerated catalyst is 0.1 wt%.

Riser reactor feedstock is light alkanes separated from the product gas flow and water vapor, with a water vapor content of 80 wt%.

Riser reactor conditions: gas superficial velocity is 10.0 m/s, temperature is 580°C, pressure is 500 kPa, bed density is 50 kg/m³.

Bed reactor feedstock is C₃, C₄, and Cs hydrocarbons separated from the product gas flow.

Light hydrocarbon aromatization reaction zone conditions: gas superficial velocity is 2.0 m/s, reaction temperature is 550°C, reaction pressure is 500 kPa, bed density is 150 kg/m³.

Single-pass conversion of naphtha feedstock into the naphtha to aromatics reactor is 74 wt%.

Product distribution: 67 wt% BTX, 11 wt% light olefins, 5.3 wt% hydrogen, 3 wt% light alkanes, 5 wt% combustible gas, 8 wt% heavy aromatics, 0.7 wt% coke. The content of p-xylene in the mixed xylene in the product is 64 wt%.

### Example 3

This embodiment employs the device illustrated in Fig. 1.

Naphtha feedstock fed to the naphtha to aromatics reactor is coal direct liquefaction naphtha with an aromatics potential content of 3 wt%, including unconverted naphtha recycled from the separated product gas flow.

Naphtha to aromatics reaction zone conditions: gas superficial velocity is 1.2 m/s, reaction temperature is 550°C, reaction pressure is 120 kPa, bed density is 260 kg/m³.

Regeneration gas is oxygen-enriched air.

Regeneration zone conditions in the regenerator: gas superficial velocity is 1.2 m/s, regeneration temperature is 650°C, regeneration pressure is 120 kPa, bed density is 260 kg/m³.

Carbon content in the regenerated catalyst is 0.3 wt%.

Riser reactor feedstock is light alkanes separated from the product gas flow and water vapor, with a water vapor content of 25 wt%.

Riser reactor conditions: gas superficial velocity is 7.0 m/s, temperature is 630°C, pressure is 120 kPa, bed density is 80 kg/m³.

Bed reactor feedstock is C₄ and C₅ hydrocarbons separated from the product gas flow.

Light hydrocarbon aromatization reaction zone conditions: gas superficial velocity is 1.2 m/s, reaction temperature is 580°C, reaction pressure is 120 kPa, bed density is 260 kg/m³.

Single-pass conversion of naphtha feedstock into the naphtha to aromatics reactor is 94.5 wt%.

Product distribution: 60 wt% BTX, 14 wt% light olefins, 7 wt% hydrogen, 8 wt% light alkanes, 5 wt% combustible gas, 5.5 wt% heavy aromatics, 0.5 wt% coke. The content of p-xylene in the mixed xylene in the product is 65 wt%.

### Example 4

This embodiment employs the device illustrated in Fig. 1.

Naphtha feedstock fed to the naphtha to aromatics reactor is coal direct liquefaction naphtha with an aromatics potential content of 46 wt%, including unconverted naphtha recycled from the separated product gas flow.

Naphtha to aromatics reaction zone conditions: gas superficial velocity is 1.8 m/s, reaction temperature is 600°C, reaction pressure is 200 kPa, bed density is 220 kg/m³.

Regeneration gas is air.

Regeneration zone conditions in the regenerator: gas superficial velocity is 1.8 m/s, regeneration temperature is 700°C, regeneration pressure is 200 kPa, bed density is 220 kg/m³.

Carbon content in the regenerated catalyst is 0.1 wt%.

Riser reactor feedstock is light alkanes separated from the product gas flow and water vapor, with a water vapor content of 50 wt%.

Riser reactor conditions: gas superficial velocity is 5.0 m/s, temperature is 660°C, pressure is 220 kPa, bed density is 110 kg/m³.

Bed reactor feedstock is C₄ and C₅ hydrocarbons separated from the product gas flow.

Light hydrocarbon aromatization reaction zone conditions: gas superficial velocity is 1.8 m/s, reaction temperature is 630°C, reaction pressure is 200 kPa, bed density is 220 kg/m³.

Single-pass conversion of naphtha feedstock into the naphtha to aromatics reactor is 88 wt%.

Product distribution: 70.2 wt% BTX, 10 wt% light olefins, 6 wt% hydrogen, 5 wt% light alkanes, 4 wt% combustible gas, 4 wt% heavy aromatics, 0.8 wt% coke. The content of p-xylene in the mixed xylene in the product is 61 wt%.

### Example 5

This embodiment employs the device illustrated in Fig. 1.

Naphtha feedstock fed to the naphtha to aromatics reactor is coal direct liquefaction naphtha with an aromatics potential content of 64 wt%, including unconverted naphtha recycled from the separated product gas flow.

Naphtha to aromatics reaction zone conditions: gas superficial velocity is 1.0 m/s, reaction temperature is 580°C, reaction pressure is 150 kPa, bed density is 350 kg/m³.

Regeneration gas is air.

Regeneration zone conditions in the regenerator: gas superficial velocity is 1.0 m/s, regeneration temperature is 680°C, regeneration pressure is 150 kPa, bed density is 350 kg/m³.

Carbon content in the regenerated catalyst is 0.5 wt%.

Riser reactor feedstock is light alkanes separated from the product gas flow and water vapor, with a water vapor content of 40 wt%.

Riser reactor conditions: gas superficial velocity is 7.0 m/s, temperature is 650°C, pressure is 150 kPa, bed density is 80 kg/m³.

Bed reactor feedstock is C₄ and C₅ hydrocarbons separated from the product gas flow.

Light hydrocarbon aromatization reaction zone conditions: gas superficial velocity is 1.0 m/s, reaction temperature is 610°C, reaction pressure is 150 kPa, bed density is 350 kg/m³.

Single-pass conversion of naphtha feedstock into the naphtha to aromatics reactor is 76 wt%.

Product distribution: 72 wt% BTX, 8 wt% light olefins, 5 wt% hydrogen, 3 wt% light alkanes, 6 wt% combustible gas, 5 wt% heavy aromatics, 1.0 wt% coke. The content of p-xylene in the mixed xylene in the product is 55 wt%.

The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A device for producing aromatics from naphtha, wherein the device for producing aromatics from naphtha comprises a naphtha to aromatics reactor, a regenerator, and a light hydrocarbon aromatization reactor;
the naphtha to aromatics reactor is connected to the regenerator via a spent catalyst delivery pipe I; the regenerator is connected to the naphtha to aromatics reactor via a regenerated catalyst delivery pipe;
the light hydrocarbon aromatization reactor includes a riser reactor, and the riser reactor is connected to a bed reactor; and
the regenerator is connected to the riser reactor via a regenerated catalyst slide valve II; the bed reactor is connected to the regenerator via a spent catalyst delivery pipe II.

2. The device for producing aromatics from naphtha according to claim 1, wherein a gas-solid separation zone is provided on the upper part of the naphtha to aromatics reactor; a product gas delivery pipe I is provided in the gas-solid separation zone; and
a naphtha to aromatics reaction zone is provided at the bottom of the naphtha to aromatics reactor; the regenerated catalyst delivery pipe inputs a catalyst into the naphtha to aromatics reaction zone; a naphtha to aromatics reactor distributor for introducing a naphtha raw material is provided at the bottom of the naphtha to aromatics reaction zone.

3. The device for producing aromatics from naphtha according to claim 2, wherein a gas-solid separation unit I and a gas collection chamber I are provided in the naphtha to aromatics reactor; the gas collection chamber I is located at the top of the gas-solid separation zone; a gas outlet of the gas-solid separation unit I is connected to the gas collection chamber I, and the gas collection chamber I is connected to the product gas delivery pipe I.

4. The device for producing aromatics from naphtha according to claim 2, wherein a stripper I is provided beneath the naphtha to aromatics reaction zone; the naphtha to aromatics reaction zone is connected to the spent catalyst delivery pipe I via the stripper I.

5. The device for producing aromatics from naphtha according to claim 4, wherein the stripper I is connected to the spent catalyst delivery pipe I via the spent catalyst slide valve I.

6. The device for producing aromatics from naphtha according to claim 3, wherein the gas-solid separation unit I adopts one or more groups of gas-solid cyclone separators, and each group of gas-solid cyclone separators includes a first-stage gas-solid cyclone separator and a second-stage gas-solid cyclone separator.

7. The device for producing aromatics from naphtha according to claim 1, wherein a regenerator gas-solid separation zone is provided on upper part of the regenerator; a flue gas delivery pipe is provided in the regenerator gas-solid separation zone;
a regeneration zone is provided at the lower part of the regenerator; the spent catalyst delivery pipe I and the spent catalyst delivery pipe II are used to feed the spent catalyst into the regeneration zone; a regenerator distributor for introducing a regeneration gas is provided at lower part of the regenerator;
the regenerated catalyst delivery pipe delivers a regenerated catalyst in the regeneration zone to the naphtha to aromatics reactor; the regenerated catalyst slide valve II delivers the regenerated catalyst to the riser reactor.

8. The device for producing aromatics from naphtha according to claim 7, wherein a regenerator gas-solid separation unit and a regenerator gas collection chamber are provided in the regenerator shell; the regenerator gas collection chamber is located at the top of the regenerator gas-solid separation zone; a gas outlet of the regenerator gas-solid separation unit is connected to the regenerator gas collection chamber; the regenerator gas collection chamber is connected to the flue gas delivery pipe.

9. The device for producing aromatics from naphtha according to claim 7, wherein a regenerator stripper is provided beneath the regeneration zone; the regeneration zone is connected to the regenerated catalyst slide valve I and the regenerated catalyst slide valve II via the regenerator stripper;
the regenerated catalyst slide valve I is connected to the naphtha to aromatics reactor via the regenerated catalyst delivery pipe; the regenerated catalyst slide valve II is connected to the riser reactor.

10. The device for producing aromatics from naphtha according to claim 8, wherein the regenerator gas-solid separation unit adopts one or more groups of gas-solid cyclone separators, and each group of gas-solid cyclone separators includes a first-stage gas-solid cyclone separator and a second-stage gas-solid cyclone separator.

11. The device for producing aromatics from naphtha according to claim 1, wherein a bed reactor gas-solid separation zone is provided on upper part of the bed reactor; a product gas delivery pipe II is provided in the bed reactor gas-solid separation zone;
a light hydrocarbon aromatization reaction zone is provided at the lower part of the bed reactor;
a bed reactor distributor is provided at the lower part of the light hydrocarbon aromatization reaction zone; the bed reactor distributor is used to feed a bed reactor feedstock; upper end of the riser reactor penetrates bottom of the bed reactor and is inserted into the bed reactor along axial direction;
the regenerated catalyst slide valve II delivers a regenerated catalyst to a feed inlet end of the riser reactor.

12. The device for producing aromatics from naphtha according to claim 11, wherein a gas-solid separation unit II and a gas collection chamber II are provided in the bed reactor gas-solid separation zone; a gas outlet of the gas-solid separation unit II is connected to the gas collection chamber II; a catalyst outlet of the gas-solid separation unit II is located in the light hydrocarbon aromatization reaction zone; the gas collection chamber II is connected to a product gas delivery pipe II located outside the bed reactor.

13. The device for producing aromatics from naphtha according to claim 11, wherein the light hydrocarbon aromatization reaction zone is connected to a stripper II, and the bed reactor is connected to the spent catalyst delivery pipe II via the stripper II.

14. The device for producing aromatics from naphtha according to claim 1, wherein the stripper II is connected to the spent catalyst delivery pipe II via the spent catalyst slide valve II.

15. The device for producing aromatics from naphtha according to claim 12, wherein the gas-solid separation unit II is a gas-solid cyclone separator; and the catalyst outlet of the gas-solid separation unit II is located above outlet end of the riser reactor.

16. A method for preparing aromatics from naphtha, using the device according to any one of claims 1 to 15; a catalyst is a metal molecular sieve bifunctional catalyst;
feeding a raw material containing naphtha into the naphtha to aromatics reactor; feeding a riser reactor feedstock into the riser reactor; feeding the bed reactor feedstock containing C₃, C₄ and C₅ hydrocarbons into the bed reactor; feeding a regeneration gas to the regenerator;
the naphtha to aromatics reactor and the bed reactor output a product gas, and a spent catalyst is input into the regenerator via the spent catalyst delivery pipe I and the spent catalyst delivery pipe II; after the spent catalyst reacts with the regeneration gas in the regenerator to be regenerated, it is fed into the naphtha to aromatics reactor and the riser reactor; the regenerator discharges a flue gas.

17. The method for producing aromatics from naphtha according to claim 16, wherein the catalyst is a metal-modified HZSM-5 zeolite molecular sieve; a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

18. The method according to claim 16, wherein the naphtha is at least one selected from the group consisting of coal direct liquefaction naphtha, coal indirect liquefaction naphtha, straight-run naphtha, and hydrocracked naphtha.

19. The method according to claim 16, wherein the raw material containing naphtha further comprises unconverted naphtha separated from the product gas flow; the unconverted naphtha contains linear aliphatic hydrocarbons, branched aliphatic hydrocarbons, and naphthenes of C₄-C₁₂.

20. The method according to claim 16, wherein the reaction conditions in the naphtha to aromatics reaction zone are: gas superficial velocity in a range from 0.5 m/s to 2.0 m/s, reaction temperature in a range from 500°C to 650°C, reaction pressure in a range from 100 kPa to 500 kPa, bed density in a range from 150 kg/m³ to 700 kg/m³.

21. The method according to claim 16, wherein the carbon content in the regenerated catalyst is ≤0.5 wt%.

22. The method according to claim 16, wherein the regeneration gas is at least one selected from the group consisting of oxygen, air, and oxygen-enriched air.

23. The method according to claim 16, wherein the regeneration conditions in the regeneration zone are: gas superficial velocity in a range from 0.5-2.0 m/s, regeneration temperature in a range from 600°C to 750°C, regeneration pressure in a range from 100 KPa to 500 kPa, bed density in a range from 150 kg/m³ to 700 kg/m³.

24. The method according to claim 16, wherein the riser reactor feedstock further comprises water vapor; the water vapor content in the riser reactor feedstock is in a range from 0 wt% to 80 wt%.

25. The method according to claim 16, wherein the light alkanes in the riser reactor feedstock are separated from the product gas flow.

26. The method according to claim 16, wherein the process conditions for the riser reactor are: gas superficial velocity in a range from 3.0 m/s to 10.0 m/s, temperature in a range from 580 to 700°C, pressure in a range from 100 to 500 kPa, bed density in a range from 50 kg/m³ to 150 kg/m³.

27. The method according to claim 16, wherein the bed reactor feedstock comprises at least one selected from the group consisting of C₃, C₄, and Cs hydrocarbons; the C₃, C₄, and Cs hydrocarbons are separated from the product gas flow.

28. The method according to claim 16, wherein the bed reactor feedstock comprises at least one selected from the group consisting of C₄ and C₅ hydrocarbons; the C₄ and C₅ hydrocarbons are separated from the product gas flow.

29. The method according to claim 16, wherein the process conditions for the light hydrocarbon aromatization reaction zone are: gas superficial velocity in a range from 0.5 m/s to 2.0 m/s, reaction temperature in a range from 550°C to 665°C, reaction pressure in a range from 100 kPa to 500 kPa, bed density in a range from 150 kg/m³ to 700 kg/m³.

30. The method according to claim 16, wherein the method comprises: introducing naphtha into the naphtha to aromatics reaction zone of the naphtha to aromatics reactor via the naphtha to aromatics reactor distributor, where it contacts with a catalyst from the regenerator to generate a product gas flow containing BTX, light olefins, hydrogen, light alkanes, combustible gas, heavy aromatics, and unconverted naphtha, while the catalyst becomes coked and converts to the spent catalyst;
feeding the regeneration gas into the regeneration zone of the regenerator via the regenerator distributor, where it contacts with the spent catalyst from the naphtha to aromatics reactor and the spent catalyst from the light hydrocarbon aromatization reactor, reacting with coke on the spent catalyst to generate flue gas and the spent catalyst converts to the regenerated catalyst;
feeding the riser reactor feedstock into the feed inlet end of the riser reactor, which contacts with the regenerated catalyst from the regenerator, converting under catalytic action into a flow containing BTX, light olefins, and H₂, which then enters the lower part of the light hydrocarbon aromatization reaction zone in the bed reactor via the outlet end of the riser reactor;
feeding the bed reactor feedstock into the light hydrocarbon aromatization reaction zone via the bed reactor distributor, where it contacts with a catalyst from the riser reactor to generate light hydrocarbon aromatization product gas flow containing BTX, light olefins, and H₂, while the catalyst becomes coked and converts to the spent catalyst.
